# EUROPEAN PATENT APPLICATION

(11) **EP 2 465 468 A1**
(43) Date of publication of application: **20.06.2012**
(21) Application number: 09848270.6
(22) Date of filing: 13.08.2009
(51) Int. Cl.: A61C 19/04, A61B 17/56, A61C 8/00

(54) **BONE CALIPERS**

(71) Applicant: Teramoto, Nobuhisa, Wakayama 642-0028 (JP)
(72) Inventor: Teramoto, Nobuhisa, Wakayama 642-0028 (JP)
(74) Representative: Emde, Eric
(86) International application number: PCT/JP2009/064293
(87) International publication number: WO 2011/018851

(57) **Abstract**

A bone caliper 1 of the present invention is used in implant treatment, and measures the width (bone width) between the bone surface of the alveolar bone and a guide hole or an implant hole. The bone caliper 1 of the present invention has a shape resembling a pair of scissors, and is constituted by a probe arm 2 having a rod-shaped measurement portion 22 formed at its front end and a bent arm 3 having a curved-shaped measurement portion 32 formed at its front end. A tip 33 of the measurement portion 32 is machined into a needle shape and opposes a tip 23 of the measurement portion 22. For measurement of the bone width, the rod-shaped measurement portion 22 of the probe arm 2 is inserted into a guide hole or an implant hole, and the tip 33 of the curved measurement portion 32 of the bent arm 3 is pressed against the gingiva until the tip 33 reaches the bone surface of the alveolar bone. In this state, the numerical value on a scale plate 6 pointed by a pointer 7 is read.

## Description

### Technical Field

The present invention relates to a bone caliper for use in measuring the bone width during implant treatment.

### Background Art

In the dental field, as a method for securely and firmly implanting an artificial tooth into a portion where a tooth is missing, implant treatment for placing an implant body has been widely performed in recent years. As used herein, implant treatment refers to a treatment in which an implant body that is an artificial tooth root is implanted into the alveolar bone so that the implant body replaces the function of a lost tooth. As materials for the implant body, a metal having high tissue affinity, such as titanium, and hydroxyapatite are used.

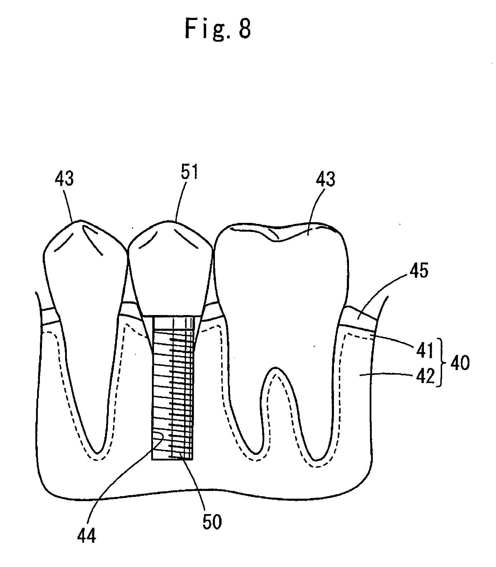

FIG. 8 shows a cross section of a relevant part of the alveolar bone in which an implant body has been placed by implant treatment. The structure of the implant body will be described briefly with reference to FIG. 8. Hatching is not shown for clarity of the drawing.

In the alveolar bone 40, a cylindrical implant hole 44 is formed in an extraction region between two teeth 43. The implant hole 44 passes through the cortical bone 41 at the surface of the alveolar bone 40 and reaches the cancellous bone 42 located inside. The outer surface of the alveolar bone 40 is covered with the gingiva 45.

The outer circumference surface of an implant body 50 is threaded, and thus by screwing the implant body 50 into the implant hole 44, the implant body 50 is firmly fixed to the alveolar bone 40.

Although described in detail later, the implant body 50 is made up of a fixture constituting a lower part and an abutment constituting an upper part, and an superstructure 51 such as a metal crown or a ceramic crown is fixed to the upper abutment by an adhesive or the like.

In the above-described implant treatment, in order to safely and securely place the implant body 50 into the alveolar bone 40, it is necessary to measure the shape of the coronal section of the alveolar bone 40 and form the implant hole 44 in an appropriate position. Forming the implant hole 44 in an inappropriate position will result in damage to a nerve leading to facial paralysis or damage to an artery that is near the surface of the alveolar bone 40 causing bleeding.

A method for measuring the shape of the coronal section of the alveolar bone 40 is use of an X-ray CT scanner (see for example, Patent Document 1). Using an X-ray CT scanner enables accurate measurement of the shape of the coronal section, but the apparatus is extremely expensive. Also, in order to form the implant hole 44 in an appropriate position, it is necessary to repeatedly check the position of a guide hole by using an X-ray CT scanner during treatment, and frequent use of the X-ray CT scanner is undesirable considering exposure to X-rays.

As a method for measuring the shape of the coronal section of the alveolar bone 40 without using an expensive apparatus such as an X-ray CT scanner, there is a method in which the gingiva is separated widely from the bone surface by an incision to expose the bone surface. This method, however, poses a physical burden on the patient and a risk of infection.

As a simple method for measuring the shape of the coronal section of the alveolar bone 40 without using an X-ray CT scanner, there is a method in which irregularities of the alveolar bone are measured by palpation or bone mapping using a gingival gauge, and the cross-sectional shape is estimated based on the result of the measurement. Although this method does not require a wide incision of the gingiva, it is less accurate in measuring the cross-sectional shape and does not eliminate the risk of damaging the nerves or arteries during implant treatment.

### Citation List

### Patent Document

Patent Document 1: JP 2003-245289A

### Summary of Invention

### Technical Problem

The present invention has been conceived in view of the above circumstances, and it is an object of the present invention to provide a bone caliper having an optimal structure as a means for checking the position of a guide hole when forming an implant hole.

### Solution to Problem

In order to achieve the above object, a bone caliper according to the present invention is a bone caliper that includes a first arm and a second arm whose respective bodies are mutually supported so as to be rotatable about a pivot and that is configured to measure an interval between tips of front ends of the first and second arms by using a scale plate and a pointer that are attached to rear portions of the bodies, the bone caliper including: a rod-shaped first measurement portion formed at the front end of the first arm; and a curved-shaped second measurement portion formed at the front end of the second arm, wherein the tip of the second measurement portion is machined into a needle shape and opposes the tip of the first measurement portion.

It is preferable that a plurality of scale marks are equidistantly provided on the first measurement portion.

The first measurement portion may be capable of being attached to and detached from the body of the first arm. Also, a spherical or teardrop-shaped protrusion may be formed at the tip of the first measurement portion.

It is preferable that the scale plate having an arc-shape is attached to the rear portion of the body of one of the first and second arms, and the pointer is attached to the rear portion of the body of the other arm in a position opposed to the scale plate. It is also preferable that rings are respectively formed at rear ends of the first and second arms.

### Advantageous Effects of Invention

With the use of the bone caliper of the present invention, it is possible to form an implant hole in an optimal position of the alveolar bone without damaging the nerves or arteries. As a result, without the use of an expensive X-ray CT scanner, implant treatment can be performed safely and securely at a low cost.

### Brief Description of Drawings

[FIG. 1] FIG.1 is a plan view of a bone caliper according to an embodiment of the present invention.
[FIG. 2] FIG. 2 is a side view of the bone caliper according to an embodiment of the present invention.
[FIG. 3] FIG. 3 is a perspective view of the bone caliper according to an embodiment of the present invention.
[FIGS. 4] FIGS. 4 are plan views showing tip portions of bone calipers whose probe arm and bent arm are differently arranged.
[FIGS. 5] FIGS. 5 are cross-sectional views illustrating the first half of a procedure for performing implant treatment using the bone caliper of the embodiment.
[FIGS. 6] FIGS. 6 are cross-sectional views illustrating the latter half of the procedure for performing implant treatment using the bone caliper of the embodiment.
[FIGS. 7] FIGS. 7 are plan views showing variations of the probe arm.
[FIG. 8] FIG. 8 is a cross-sectional view of a relevant part of the alveolar bone in which an implant body has been placed.

### Description of Embodiments

Hereinafter, a bone caliper according to an embodiment of the present invention will be described with reference to the drawings.

### Structure of Bone Caliper

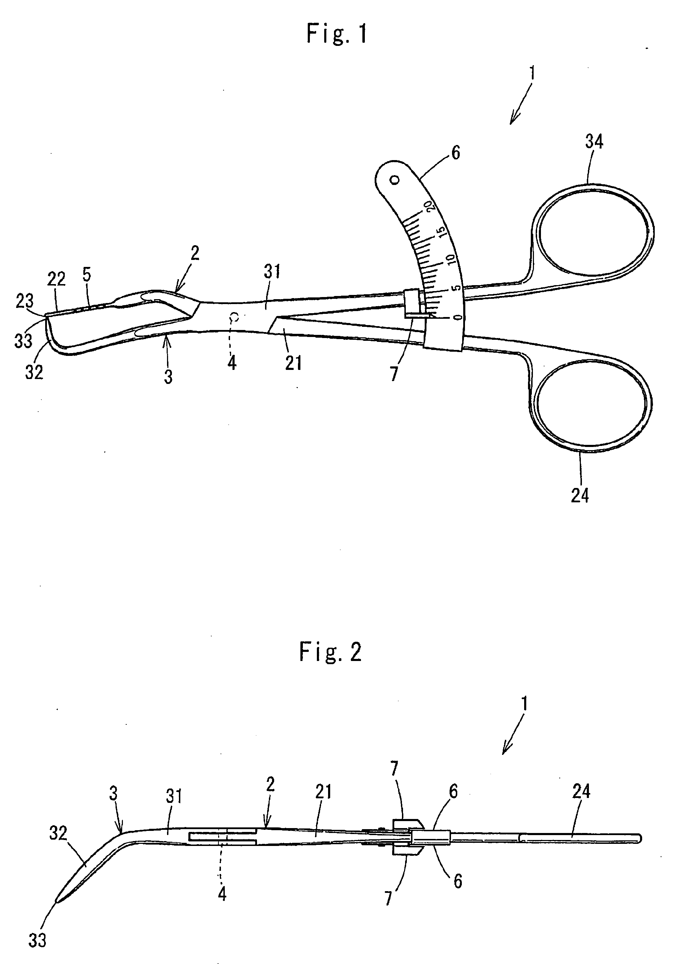

FIGS. 1, 2 and 3 respectively show a plan view, a side view and a perspective view of a bone caliper 1 according to the present embodiment. The bone caliper 1 has a shape resembling a pair of scissors, and is configured such that a probe arm 2 that is a first arm can be rotated about a pivot 4 relative to a bent arm 3 that is a second arm.

The bone caliper 1 is a tool for measuring the width of bone sandwiched between tips of measurement portions provided at front ends of the probe arm 2 and the bent arm 3 by using a scale plate 6 and a pointer 7 that are attached to rear portions of the respective arms.

The probe arm 2 is constituted by a body 21 supported so as to be rotatable about the pivot 4, a thin rod-shaped measurement portion 22 formed at its front end, and a ring 24 formed at its rear end. A plurality of ring-shaped grooves that function as scale marks 5 are formed equidistantly around the outer circumference surface of the rod constituting the measurement portion 22.

The bent arm 3 is constituted by a body 31 to which the pivot 4 is fixed, a measurement portion 32 formed in a curved shape at its front end, and a ring 34 formed at its rear end. The measurement portion 32 has a tip 33 formed in a needle shape, and the tip 33 opposes a tip 23 of the measurement portion 22 of the probe arm 2. The pivot 4 is formed integrally with the body 31 of the bent arm 3, and is inserted through an aperture formed in the body 21 of the probe arm 2.

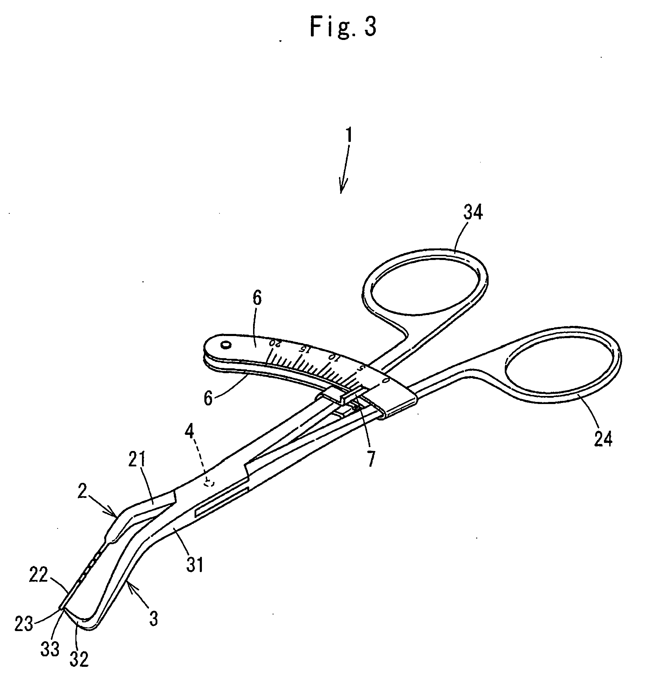

As shown in FIGS. 2 and 3, the measurement portion 22 of the probe arm 2 and the measurement portion 32 of the bent arm 3 are angled with respect to the bodies 21 and 31 in order to facilitate insertion into an oral cavity and measurement of the bone width of the alveolar bone.

The arc-shaped scale plate 6 is attached to a rear portion of the body 21 of the probe arm 2 so as to extend in a direction perpendicular to the body 21. Also, the pointer 7 is attached to a rear portion of the body 31 of the bent arm 3 in a position opposed to the scale plate 6. When the bent arm 3 is opened relative to the probe arm 2, the pointer 7 moves relative to the scale plate 6. The numerical value on the scale plate 6 pointed by the pointer 7 indicates the interval, or in other words, the linear distance between the tip 23 of the measurement portion 22 and the tip 33 of the measurement portion 32.

The scale plate 6 and the pointer 7 are provided on each of upper and lower sides of the bone caliper 1 so that the numerical value pointed by the pointer 7 can be checked from both upper and lower directions.

In the present embodiment, the scale plate 6 is attached to the body 21 of the probe arm 2, and the pointer 7 is attached to the body 31 of the bent arm 3. However, a configuration is also possible in which the pointer 7 is attached to the body 21 of the probe arm 2, and the scale plate 6 is attached to the body 31 of the bent arm 3.

The above-described bone caliper 1 of the present invention is used in implant treatment and measures the bone width, which is the interval between the bone surface of the alveolar bone and a guide hole or an implant hole.

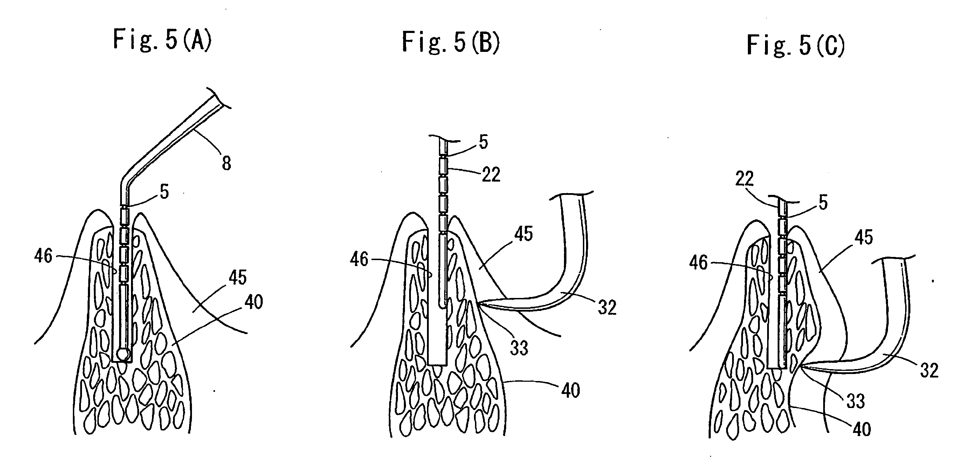

For measurement, first, the bone caliper 1 is held by the thumb and middle finger being put into the rings 24 and 34. Next, the rod-shaped measurement portion 22 of the probe arm 2 is inserted into a guide hole or an implant hole, and the tip 33 of the measurement portion 32 of the bent arm 3 is pressed against the gingiva until the tip 33 reaches the bone surface of the alveolar bone. In this state, the numerical value on the scale plate 6 pointed by the pointer 7 is read. The read numerical value is the bone width (distance) of the alveolar bone sandwiched by the tip 23 of the probe arm 2 and the tip 33 of the bent arm 3. A specific method of use of the bone caliper 1 will be described in detail later with reference to FIG. 5.

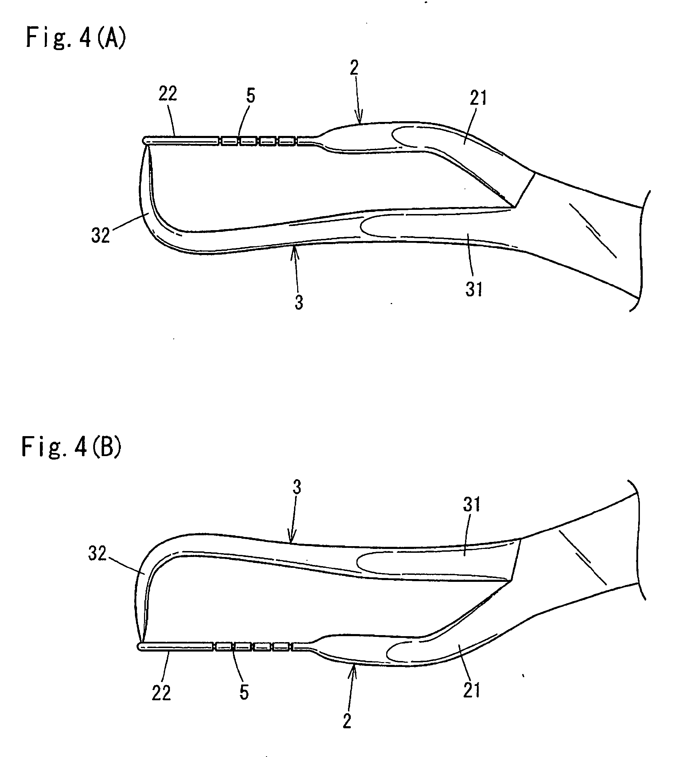

FIGS. 4(A) and 4(B) show front portions of bone calipers 1 whose probe arm 2 and bent arm 3 are differently arranged. In FIG. 4(A), as in FIG. 1, the bent arm 3 is arranged on the left side of the probe arm 2. In contrast, in FIG. 4(B), the bent arm 3 is arranged on the right side of the probe arm 2. For implant treatment, bone calipers 1 having the shapes shown in FIGS. 4(A) and 4(B) are prepared and selectively used depending on the location where the bone width is measured.

### Procedure for Implant Treatment

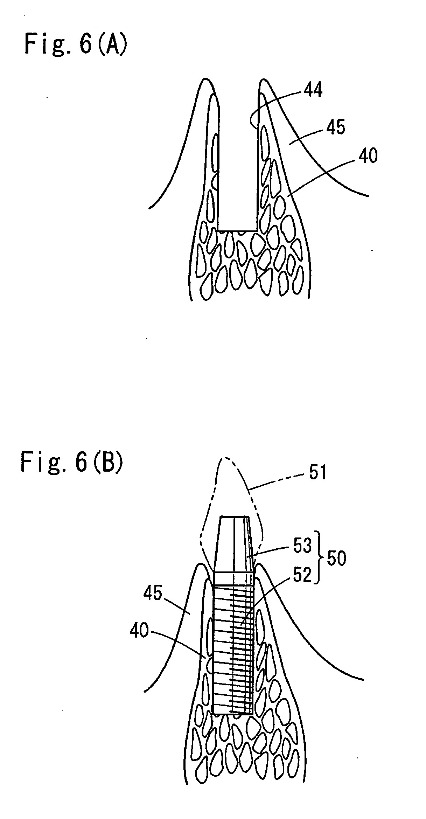

A procedure for performing implant treatment using the bone caliper 1 of the present embodiment will be described next with reference to FIGS. 5 and 6. FIGS. 5(A) to 5(C) are cross-sectional views illustrating the first half of a procedure for implant treatment. FIGS. 6(A) and 6(B) are cross-sectional views illustrating the latter half of the procedure for implant treatment. In these diagrams, the same reference numerals are given to parts that are the same as in the diagrams that have been described previously, and descriptions thereof are not given here.

Normally, before implant treatment is performed, bone tissue is built in a tooth root holder portion of the alveolar bone 40 that is hollow as a result of the removal of a tooth, by using guided bone regeneration (GBR) or the like. In the alveolar bone 40 shown in FIG. 5(A), tissue has been built in advance by GBR or the like, or bone tissue has been formed by natural healing.

Firstly, a guide hole having a diameter of approximately 1 mm is formed in the alveolar bone 40 by using a guide drill (not shown). FIG. 5(A) shows a state in which a guide hole 46 has been formed in the alveolar bone 40.

Subsequently, as shown in FIG. 5(A), a depth gauge 8 is inserted into the guide hole 46 to check whether the guide hole 46 has been formed to a predetermined depth. The depth gauge 8 has a tip portion in which scale marks 5 in the form of ring-shaped grooves are formed, as with the measurement portion 22 of the probe arm 2, so that the depth of the guide hole 46 can be measured by counting the number of scale marks 5 exposed from the guide hole 46.

Next, as shown in FIG. 5(B), the bone width of each portion of the guide hole 46 is measured by using the bone caliper 1. Specifically, in a state in which the rod-shaped measurement portion 22 of the probe gauge 2 having a diameter of approximately 0.9 mm is inserted into the guide hole 46, the tip 33 of the measurement portion 32 of the bent arm 3 is caused to penetrate the gingiva 45 and pressed against the gingiva 45 until the tip 33 reaches the bone surface of the alveolar bone 40. In this state, the numerical value on the scale plate 6 pointed by the pointer 7 is read.

FIG. 5(C) shows a case in which the guide hole 46 has been formed in an undercut portion (a portion in which the lower width is smaller than the upper width) of the alveolar bone 40. When the bone width of the alveolar bone 40 is measured, the tip 33 of the bent arm 3 is moved toward the probe arm 2 from the direction perpendicular to the probe arm 2. At this time, because the measurement portion 32 of the bent arm 3 has a curved shape, even when a depressed portion of the bone such as the undercut portion is measured, the movement thereof is not obstructed by a protruded portion of the bone.

Bone width measurement is repeatedly performed by using the two types of bone calipers 1 shown in FIGS. 4(A) and 4(B) and varying the insertion depth of the measurement portion 22 of the probe arm 2, and whether the guide hole 46 has been formed in an appropriate position is checked.

As used herein, the case where the position of the guide hole 46 is not appropriate refers to a case where a nerve passes in close proximity, and the nerve may be damaged when an implant hole 44 having a large diameter is formed in that position, or a case as shown in FIG. 5(C) where the width of the alveolar bone 40 is small, and there is a possibility that the tip of a drill might penetrate and break the wall of the alveolar bone 40, causing damage to the arteries, when an implant hole 44 is formed in that position.

After that, an operation of expanding the opening of the guide hole 46 using a larger-sized drill is repeated. Each time the operation has been performed, the bone width of each portion of the guide hole 46 is measured with the bone caliper 1, and the position and inclination of the guide hole 46 are checked. The position and inclination of the guide hole 46 are corrected based on the result of the measurement. Finally, an implant hole 44 is formed in an appropriate position by using an implant drill. FIG. 6(A) shows the thus-formed implant hole 44.

As described above, the position and inclination of the guide hole 46 are repeatedly corrected with an analog feel while checking the bone width of each portion of the guide hole 46 by using the bone caliper 1, whereby the implant hole 44 can be formed in an optimal position without damaging the nerves or arteries.

Next, as shown in FIG. 6(B), a fixture 52 for an implant body 50 is placed into the implant hole 44 formed in the above-described manner by using a ratchet or a round driver. The cylindrical fixture 52 has a diameter slightly larger than the diameter of the implant hole 44, and its outer circumference surface is threaded. Accordingly, when the fixture 52 is screwed into the implant hole 44 with a ratchet or a round driver, the fixture 52 is firmly fixed to the implant hole 44.

After the fixture 52 placed into the implant hole 44 has been completely bound to the surrounding bone tissue, a truncated conical abutment 53 is attached on top of the fixture 52. Although not shown, the abutment 53 has a small-diameter bolt fixedly attached to its underside. By screwing the bolt into a nut formed in the top of the fixture 52 near the central axis thereof, the abutment 53 is firmly fixed to the fixture 52.

Subsequently, as shown in FIG. 6(B), an superstructure 51 indicated by a phantom line is attached to the abutment 53 with an adhesive or the like. The implant treatment is thus completed.

### Variations of Probe Arm

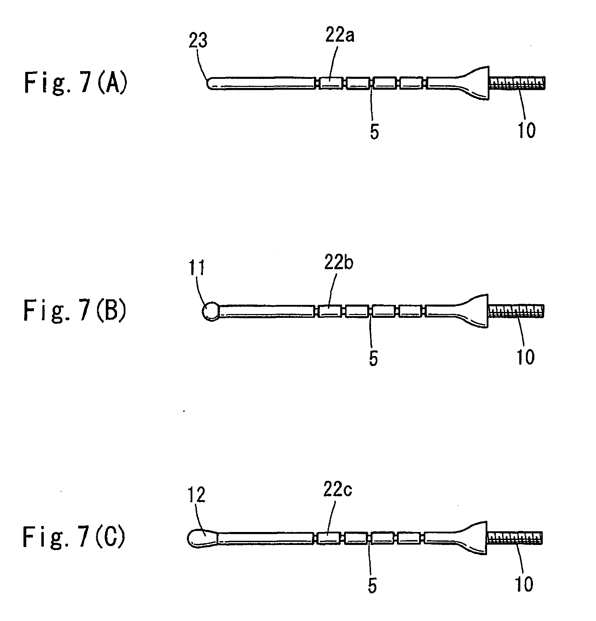

FIGS. 7 show variations of the probe arm 2. A measurement portion 22a shown in FIG. 7(A) is a variation of the measurement portion 22 of the probe arm 2 shown in FIG. 1 and configured to be removable from the body 21, and can be replaced by a measurement portion 22b shown in FIG. 7(B) or a measurement portion 22c shown in FIG. 7(C) according to the intended use. The measurement portion 22b shown in FIG. 7(B) has a spherical protrusion 11 attached to its tip. The measurement portion 22c shown in FIG. 7(C) has a teardrop-shaped protrusion 12 attached to its tip.

As shown in FIGS. 7(A) to 7(C), the measurement portions 22a to 22c for the probe arm 2 each have a small-diameter bolt 10 fixedly attached to the rear end. By screwing the bolt 10 into a nut formed at the tip of the body 21 of the probe arm 2, the measurement portions 22a to 22c can be fixed to the body 21 of the probe arm 2.

The cancellous bone 42 of the alveolar bone 40 has a low bone density, and therefore a situation may occur in which a small-diameter cavity that is in communication with the guide hole 46 is created when forming the guide hole 46. When the depth of the guide hole 46 that is in communication with the cavity is measured using the probe arm 2 having the thin measurement portion 22a shown in FIG. 7(A), the tip 23 of the measurement portion 22a enters the cavity, which causes an error in measurement.

In the case where the occurrence of such a situation is anticipated, measurement is performed using the arm having a spherical protrusion 11 attached to its tip as shown in FIG. 7(B) or the arm having a teardrop-shaped protrusion 12 attached to its tip as shown in FIG. 7(B). Because the tip 23 of the probe arm 2 will not enter the cavity that is in communication with the guide hole 46, the depth of the guide hole 46 can be accurately measured.

In FIGS. 7, the arm having a spherical protrusion 11 attached to its tip and the arm having a teardrop-shaped protrusion 12 attached to its tip were described as being removable from the body 21 of the probe arm 2, or in other words, capable of being attached to and detached from the body 21 of the probe arm 2. However, these arms may be formed integrally with the body 21. In this case, a plurality of bone calipers 1 having arm tips of different shapes need to be produced, which will increase the cost, but there is no need to replace the arm, and therefore time can be saved.

As described above, when implant treatment is performed using the bone caliper 1 of the present embodiment, the position where the implant hole 44 has been formed can be checked any time, accidents such as damaging the nerves or arteries with a drill can be prevented from occurring. Furthermore, use of an expensive apparatus such as an X-ray CT scanner is not required, and thus there is no exposure to X rays. Accordingly, implant treatment can be performed safely and at low cost.

The embodiment given above has been described in the case where the bone caliper is used in dental implant treatment, but the bone caliper of the present invention is not limited to such application, and can of course be applied to orthopedic implant treatment and plastic surgery implant treatment.

### Industrial Applicability

The bone caliper according to the present invention can be widely used in various types of implant treatments as a means for measuring the bone width between the bone surface and a guide hole or an implant hole. Reference Signs List

- 1: Bone Caliper
- 2: Probe Arm
- 3: Bent Arm
- 4: Pivot
- 5: Scale Mark
- 6: Scale Plate
- 7: Pointer
- 8: Depth Gauge
- 10: Bolt
- 11, 12: Protrusion
- 21,31: Body
- 22, 32: Measurement Portion
- 24,34: Ring
- 40: Alveolar Bone
- 44: Implant Hole
- 45: Gingiva
- 46: Guide Hole
- 50: Implant Body
- 51: Superstructure
- 52: Fixture
- 53: Abutment

## Claims

1. A bone caliper that includes a first arm and a second arm whose respective bodies are mutually supported so as to be rotatable about a pivot and that is configured to measure an interval between tips of front ends of the first and second arms by using a scale plate and a pointer that are attached to rear portions of the bodies, the bone caliper comprising:
a rod-shaped first measurement portion formed at the front end of the first arm; and
a curved-shaped second measurement portion formed at the front end of the second arm,
wherein the tip of the second measurement portion is machined into a needle shape and opposes the tip of the first measurement portion.

2. The bone caliper according to claim 1,
wherein a plurality of scale marks are equidistantly provided on the first measurement portion.

3. The bone caliper according to claim 1,
wherein the first measurement portion is capable of being attached to and detached from the body of the first arm.

4. The bone caliper according to claim 1,
wherein a spherical or teardrop-shaped protrusion is formed at the tip of the first measurement portion.

5. The bone caliper according to claim 1,
wherein the scale plate having an arc-shape is attached to the rear portion of the body of one of the first and second arms, and the pointer is attached to the rear portion of the body of the other arm in a position opposed to the scale plate.

6. The bone caliper according to claim 1,
wherein rings are respectively formed at rear ends of the first and second arms.
